(19) Europäisches Patentamt
European Patent Office
Office européen des brevets

(11) Numéro de publication: **0 072 325**
**A1**

(12) # DEMANDE DE BREVET EUROPEEN

(21) Numéro de dépôt: **82401481.5**

(22) Date de dépôt: **05.08.82**

(51) Int. Cl.³: **C 08 F 218/16**, C 07 C 69/96
// (C08F218/16, 218/16, 218/16)

(30) Priorité: **06.08.81 FR 8115295**

(43) Date de publication de la demande: **16.02.83**
**Bulletin 83/7**

(84) Etats contractants désignés: **AT BE CH DE FR GB IT LI LU NL SE**

(71) Demandeur: **SOCIETE FRANCAISE D'ORGANO-SYNTHESE Société Anonyme dite:,**
**114, avenue Louis-Roche, F-92234 Gennevilliers (FR)**

(72) Inventeur: **Pellegrina, Michel, 26 Bd Lenine, F-95100 Argentueil (FR)**
Inventeur: **Proux, Yves, 39 rue des Cevennes, F-75015 Paris (FR)**

(74) Mandataire: **Combe, André et al, CABINET BEAU DE LOMENIE 55 rue d'Amsterdam, F-75008 Paris (FR)**

(54) **Nouveau mélange polymérisable à base de bis(allylcarbonate) de diéthylèneglycol, son procédé de préparation et son utilisation pour la préparation de produits finis.**

(57) La présente invention concerne de nouveaux mélanges polymérisables à base de bis(allylcarbonate) de diéthylèneglycol, caractérisés en ce qu'ils comportent

75 à 83% en poids de bis(allylcarbonate) de diéthylèneglycol,

11 à 16% en poids de dimère de ce bis(allylcarbonate) de diéthylèneglycol,

1 à 3% en poids de trimère de ce bis(allylcarbonate) de diéthylèneglycol, et

0 à 7% en poids de polymères ou copolymères de bis(allylcarbonate) de diéthylèneglycol,

lesdits mélanges contenant

– moins de 0,1% d'eau

– moins de 1% d'ester monosubstitué

– elle concerne également un procédé de préparation, par transestérification desdits mélanges, et l'utilisation de ces mélanges pour la réalisation, par polymérisation, d'objets utilisables en optique.

EP 0 072 325 A1

Nouveau mélange polymérisable à base de bis(allylcarbonate) de diéthylèneglycol, son procédé de préparation et son utilisation pour la préparation de produits finis.

La présente invention concerne un nouveau mélange polymérisable à base de bis(allylcarbonate) de diéthylèneglycol; elle concerne aussi un procédé de préparation dudit mélange et les applications de ce mélange à la production de polymère en forme.

Le bis(allylcarbonate) de diéthylèneglycol est un monomère connu et largement utilisé notamment pour la préparation de produits en forme ayant de bonnes propriétés optiques. Cependant la préparation de ce produit, d'une part, et sa polymérisation, d'autre part, posent un certain nombre de problèmes techniques auxquels la présente invention vise à remédier.

Si l'on considère tout d'abord le problème de la polymérisation du bis(allylcarbonate) de diéthylèneglycol il a été trouvé que, contrairement à certains enseignements, il était souhaitable de soumettre à cette polymérisation non pas le monomère pur mais un mélange présentant les caractéristiques suivantes:

- être exempt d'eau (teneur en eau inférieure à 0,1 %);
- contenir une proportion aussi faible que possible d'ester monosubstitué (dans ce qui suit on appellera ester monosubstitué le produit de réaction d'une mole de diéthylèneglycol sur une mole de carbonate d'allyle de telle sorte qu'une seule fonction alcool du diéthylèneglycol soit combinée pour donner un ester. Cette quantité d'ester monosubstituée devant être inférieure à 1 % dans le mélange;
- contenir des quantités relatives précises de monomère [bis(allylcarbonate) de diéthylèneglycol] et de dimère et de trimère de ce monomère.

De tels mélanges présentent en effet, sur le plan de la viscosité, de la vitesse de polymérisation et des propriétés des polymères obtenus, un ensemble de propriétés remarquables qui en font un mélange polymérisable particulièrement utile.

La présente invention concerne donc tout d'abord des nouveaux mélanges polymérisables à base de bis(allylcarbonate) de diéthylèneglycol caractérisés en ce qu'ils comportent

de 75 à 83 % en poids de bis(allylcarbonate)

de diéthylèneglycol,

de 11 à 16 % en poids de dimère de bis (allylcarbonate) de diéthylèneglycol,

de 1 à 3 % en poids de trimère de bis (allylcarbonate) de diéthylèneglycol, et

de 0 à 7 % en poids de polymères ou copolymères de bis (allylcarbonate) de diéthylèneglycol;

de plus lesdits mélanges devront comporter

-moins de 0,1 % d'eau

-moins de 1 % d'ester monosubstitué.

Le mélange selon l'invention est essentiellement caractérisé par la présence d'une quantité relativement importante de dimère; il en résulte, d'une part, une viscosité acceptable pour les opérations de polymérisation en moule qui sont généralement mises en oeuvre lors de l'utilisation de ce mélange et, d'autre part, des propriétés intéressantes pour ce qui concerne les polymères obtenus à partir de ces mélanges.

De plus la quantité d'eau contenue dans le mélange a une influence néfaste sur la dureté du polymère final obtenu.

Enfin la quantité d'ester monosubstitué du mélange doit être limitée et controlée, d'une part parce que la présence de fonctions alcools libres dans le mélange a une influence sur le poids moléculaire de polymére final et d'autre part parce que la présence de cet ester diminue la dureté dudit polymère final.

Comme les résultats obtenus dans l'analyse de la composition de mélange selon l'invention peuvent dépendre, dans une certaine mesure, de la méthode d'analyse utilisée, on décrit ci-après la méthode employée dans la présente demande.

Le mélange est analysé par la méthode dite de chromatographie liquide, haute pression, par perméation de gel. Le solvant est le tétrahydrofuranne pur. La solution du mélange à analyser, présentant une concentration de 10 g/1, est filtrée sur une membrane en polytétrafluoroéthylène (Téflon) de 0,2 micron, cette solution est ensuite envoyée, à l'aide d'une pompe fonctionnant à 45 bars, vers un injecteur à boucle; l'analyse est effectuée à l'aide d'un

détecteur réfractomètre différentiel d'une sensibilité $2.10^{-5}$ unités I.R.; les colonnes de séparation sont au nombre quatre et sont remplies de billes de gel poreux rigide de copolymère réticulé styrène-divinylbenzène, le diamètre des pores des billes étant 100 Å pour les trois premières colonnes et 500 Å pour la quatrième; le fractionnement effectué à 25°C est obtenu par l'élution de la solution sur le gel utilisé.

Cette méthode permet de séparer :

-le bis(allylcarbonate) de diéthylèneglycol : temps de rétention 41,5 min

-le dimère de ce produit : temps de rétention 38 min

-le trimère de ce produit : temps de rétention 35,8 min

-les polymères de ce produit : temps de rétention 29 min

-enfin l'ester monosubstitué a un temps de rétention de 43,5 min.

L'eau du mélange a été analysée par la méthode FISHER.

La présente invention concerne également un procédé de préparation des mélanges polymérisables définis ci-dessus.

Jusqu'à ce jour, les principaux procédés connus de synthèse du bis(allylcarbonate) de diéthylèneglycol visaient essentiellement à la préparation d'un monomère pur. Ces procédés pouvaient se résumer comme suit :

-réaction du chloroformiate de diéthylèneglycol sur l'alcool allylique;

-réaction du chloroformiate d'allyle sur le diéthylèneglycol;

-réaction du chlorure d'allyle avec le diéthylèneglycol en présence de carbonate de sodium sous pression de gaz carbonique en présence de triéthylamine.

Ces procédés permettent, lorsqu'ils sont couplés avec des procédés connus d'isolement et de purification convenables, de préparer du monomère (à savoir le bis(allylcarbonate) de diéthylèneglycol) à l'état pur - ce qui est généralement demandé par les utilisateurs - ou conduisent à des mélanges qui peuvent contenir de 3 à 6 % de dimère de ce monomère. Le procédé selon la

présente invention - mettant en oeuvre un principe totalement différent des principes des procédés connus - permet également, lorsqu'il est couplé avec des procédés d'isolement et de purification connus, de préparer du monomère pur ou du monomère comportant une faible fraction de dimère, mais ce procédé permet essentiellement de préparer directement les mélanges selon l'invention, ces mélanges étant, sans purification ultérieure, utilisables pour la polymérisation.

Le nouveau procédé selon la présente invention consiste à réaliser une réaction de transestérification en faisant réagir du carbonate d'allyle de formule

$$CH_2=CH-CH_2-O-\overset{O}{\overset{\|}{C}}-O-CH_2-CH=CH_2$$

sur le diéthylèneglycol de formule

$$HO-CH_2-CH_2-O-CH_2-CH_2-OH.$$

Cette réaction (transestérification) est de type connu et on pouvait prévoir qu'en mettant en oeuvre une réaction de ce type on pourrait obtenir, avec un certain rendement, le produit monomère recherché. Cependant il était impossible de prévoir que, par le choix de conditions expérimentales précises pour réaliser cette transestérification, on pourrait réaliser une réaction permettant :

- d'éviter les diverses réactions secondaires prévisibles;
- de donner naissance directement aux mélanges selon l'invention, mélanges qui sont directement utilisables pour effectuer la polymérisation dans de bonnes conditions et en donnant naissance à des produits de valeur.

Les conditions opératoires à respecter sont les suivantes :

a) Utilisation d'un fort excès de carbonate d'allyle par rapport à la stoechiométrie prévisible de la réaction impliquant l'utilisation de 2 moles de carbonate d'allyle sur 1 mole de diéthylèneglycol. Il convient d'utiliser au moins 8 (et de préférence au moins 10) moles de carbonate pour 1 mole

de diéthylèneglycol. Si l'excès de carbonate est insuffisant les mélanges obtenus comportent des proportions trop importantes de dimère, de trimère et de polymères ce qui rend ces mélanges inaptes à une polymérisation directe.

On notera que le diéthylèneglycol étant peu soluble dans le carbonate d'allyle, le fait de mettre un excès de carbonate permet à ce produit de jouer le rôle de "milieu de réaction" pour la réalisation d'une réaction homogène.

b) Elimination continue de l'alcool allylique formé au cours de la réaction; cette élimination, qui s'effectue par exemple par distillation, est nécessaire pour éviter un certain nombre de réactions secondaires qui se développent en présence de cet alcool insaturé. Pour faciliter l'élimination de cet alcool allylique, il est possible d'ajouter au milieu réactionnel un composé qui formera avec l'alcool un mélange azéotrope. On utilisera par exemple le toluène (à raison d'environ 4 moles par mole de diéthylèneglycol) qui, avec l'alcool allylique, donne un mélange azéotrope 50/50 qui bout à 91-92°C (vapeur).

c) Utilisation d'un catalyseur convenable de transestérification. Les catalyseurs de transestérification sont nombreux. Il a été trouvé selon la présente invention que les catalyseurs à utiliser devaient être choisis parmi les esters de l'acide titanique (tel que par exemple le titanate de n.butyle) et les acétylacétonates métalliques (plus particulièrement les acétylacétonates de titane ou de zirconium).

De plus ces catalyseurs devront être utilisés en quantités convenables à savoir entre $0,2 . 10^{-2}$ et $2.10^{-2}$ mole de catalyseur par mole de diéthylèneglycol.

d) Emploi d'une température adéquate. La température a une influence certaine sur le rendement et la spécificité de la réaction de transestérification. Si la température est insuffisante, on constate, d'une part, l'exixtence d'un rendement de réaction insuffisant et, d'autre part, l'absence du di- et du trimère qui sont souhaités dans le cadre de la présente invention. Compte tenu de ce que la température d'ébullition du carbonate d'allyle est de 167°C, il a été trouvé que la température de réaction

devait se situer entre 110 et 160°C et de préférence (selon le catalyseur) entre 130 et 155°C.

On notera de plus que grâce à un choix convenable des températures utilisées il est possible de faire en sorte que la teneur du mélange en ester monosubstitué soit aussi faible que possible. Pour cela il conviendra de choisir, en fin de réaction, une température relativement basse (110-130°C par exemple) et de diminuer alors la pression au-dessus du mélange réactionnel de façon à faciliter la distillation de l'alcool allylique présent dans le mélange.

La présente invention concerne donc un procédé de préparation de bis(allylcarbonate) de diéthylèneglycol et des mélanges contenant notamment 11 à 16 % en poids de dimère, 1 à 3 % en poids de trimère et 0 à 7 % en poids de polymère de ce produit, lesdits mélanges contenant moins de 0,1 % d'eau et moins de 1 % d'ester monosubstitué et étant directement utilisables pour la polymérisation, ledit procédé consistant dans une réaction de transestérification entre le carbonate d'allyle et le diéthylèneglycol, la réaction étant effectuée sous atmosphère inerte et en présence d'un excès de carbonate d'allyle et étant caractérisée en ce que

- la température de réaction est choisie entre 110 et 160°C, de préférence entre 130 et 155°C, avec élimination continue de l'alcool allylique formé, ladite élimination s'effectuant, en fin de réaction à une température de 110 à 130°C et sous un vide partiel.
- le catalyseur, utilisé à raison de $0,2.10^{-2}$ à $2.10^{-2}$ mole par mole de diéthylèneglycol, est choisi parmi les esters de l'acide titanique et les acétylacétonates métalliques,

puis, après élimination du catalyseur, on distille l'excès de carbonate pour récupérer le mélange polymérisable recherché.

La présente invention concerne enfin l'application des nouveaux mélanges polymérisables à la préparation de produits solides en forme, notamment des produits utilisables en optique, par polymérisation de ces mélanges à l'aide de techniques connues précédemment décrites pour réaliser la polymérisation du bis(allylcarbonate) de diéthylèneglycol pur.

Les exemples non limitatifs suivants illustrent l'invention.

Exemple 1

Dans un réacteur en verre Pyrex de 4 litres muni d'un agitateur, d'un thermomètre et d'un appareil de condensation (utile pour l'évacuation de l'alcool allylique) on a introduit 2840 g (20 moles) de carbonate d'allyle pur et 4,1 g ($1,2 \times 10^{-2}$ mole) de titanate de n-butyle. L'appareil est purgé à l'azote (un bullage de ce gaz étant maintenu pendant les essais) avant de porter la masse à la température T, température à laquelle on ajoute en 30 min 212 g (2 moles) de diéthylèneglècol de haute pureté.

La température est maintenue entre T et T + 5°C durant 2 heures sous la pression appropriée permettant l'élimination progressive de l'alcool allylique formé puis, après refroidissement jusque vers 100°C et retour à la pression atmosphérique (sous azote) lorsque cela est nécessaire le catalyseur est détruit par hydrolyse. Le précipité obtenu est séparé par filtration et le mélange restant est concentré sous pression réduite (3 torr) sans dépasser 105 à 110°C; cela en présence de charbon actif. On élimine ainsi l'excès de carbonate d'allyle. Le produit fitré est soumis à l'analyse par chromatographie liquide haute pression décrite précédemment.

Suivant la température T utilisée les résultats suivants ont été obtenus :

| T(°C) | 155 | 130 | 105 | 80 |
|---|---|---|---|---|
| | | | | (diéthylèneglycol 4,1) |
| $CH_2=CH-CH_2CO_3CH_2CH_2OCH_2CH_2OH$ | 2,2 | 5,2 | 3 | 73,7 |
| bis(allylcarbonate) de diéthylèneglycol | 78,4 | 75,4 | 61,2 | 9,2 |
| $CH_2=CH-CH_2 -(CO_3CH_2CH_2OCH_2CH_2)_2OH$ | 0 | 0,9 | 0,7 | 0,6 |
| dimère du bis(allylcarbonate) de diéthylèneglycol | 12,9 | 11,7 | 20,9 | 0 |
| trimère - | 1,8 | 1,0 | 6,3 | 0 |
| polymères | 4,7 | 5,5 | 7,9 | 12,4 |
| $R= \frac{\text{poids recueilli x 100}}{2 \times 274,3}$ (%) | 95,6 | 92,7 | 93,8 | 22,6 |

Dans le tableau ci-dessus (comme pour les exemples qui suivent) le rendement représente la quantité totale de produit isolé par rapport à la stoechiométrie de la réaction en bis(allylcarbonate) de diéthylèneglycol pur basée sur le diéthylèneglycol engagé.

Ces résultats montrent qu'au-dessous d'environ 100°C la réaction ne se produit que très partiellement et qu'à 105°C la sélectivité des produits recherchés n'est pas bonne, Au-delà nous obtenons les mélanges visés.

Exemple 2

On a reproduit l'essai de l'exemple 1 à 155°C en :

remplaçant le titanate de n-butyle par l'acétylacétonate de zirconium toujours à raison de 0,6 x $10^{-2}$ mole/mole de diéthylèneglycol. La composition du produit isolé est la suivante :

| | |
|---|---|
| $CH=CH-CH_2CO_3CH_2CH_2OCH_2CH_2OH$ | 2,7 % |
| bis(allyl carbonate) de diéthylèneglycol | 79,0 % |
| $CH_2=CH-CH_2(CO_3CH_2CH_2OCH_2CH_2)_2OH$ | 0 % |
| dimère du bis(allylcarbonate) de diéthylèneglycol | 14,4 % |
| trimère - | 2,4 % |
| polymères | 1,5 % |

Elle est tout à fait comparable à celle du mélange obtenu dans les mêmes conditions avec le titanate de n-butyle (et le rendement est équivalent).

Exemple 3

On a reproduit l'essai de l'exemple 1 en se plaçant à la température de 155°C et en utilisant comme catalyseur le titanate de n.butyle dont nous avons fait varier le taux de 0,14 mole/mole de diéthylèneglycol à 2 x $10^{-2}$ mole/mole de diéthylèneglycol.

Les résultats suivants ont été observés :

| Taux de catalyseur (mole/mole de diéthylèneglycol) | $0,14x10^{-2}$ | $0,2x10^{-2}$ | $0,6x10^{-2}$ | $0,75x10^{-2}$ | $1x10^{-2}$ | $2x10^{-2}$ |
|---|---|---|---|---|---|---|
| $CH_2=CH-CH_2CO_3CH_2CH_2OCH_2CH_2OH$ | 3,8 | 3,2 | 0,6 | 4,3 | 2,8 | 0,5 |
| bis(allylcarbonate) de diéthylèneglycol | 68,0 | 75,7 | 80,1 | 78,5 | 80,8 | 78,5 |
| $CH_2=CH-CH_2(CO_3CH_2CH_2OCH_2CH_2)_2OH$ | 0 | 0,6 | 0,2 | 0 | 0 | 0 |
| dimère du bis(allylcarbonate) de diéthylèneglycol | 9,8 | 14,1 | 15,1 | 12,5 | 12,7 | 16,5 |
| trimère — | 1,2 | 2,3 | 2,3 | 1,2 | 1,0 | 2,5 |
| polymères | 17,2 | 4,7 | 1,7 | 1,2 | 2,7 | 2,0 |

Lorsqu'il n'y a pas de catalyseur on n'obtient qu'un solide blanc dont la partie soluble dans le tétrahydrofuranne a pour masse moléculaire moyenne en poids 20 000 et pour masse moléculaire moyenne en nombre 7 000 (indice de dispersion : 6,8 ).

Exemple 4

On a reproduit l'essai de l'exemple 1 réalisé à la température de 155°C en faisant varier l'excès de carbonate d'allyle par rapport au diéthylèneglycol. Les compositions suivantes ont été obtenues :

| Rapport molaire carbonate d'allyle/diéthylèneglycol | 6/1 | 8/1 | 10/1 | 12/1 |
|---|---|---|---|---|
| $CH_2=CH-CH_2CO_3CH_2CH_2OCH_2CH_2OH$ | 7,1 | 4,6 | 2,2 | 4,5 |
| bis(allylcarbonate) de diéthylèneglycol | 61,0 | 76,5 | 78,4 | 75,6 |
| $CH_2=CH-CH_2-(CO_3CH_2CH_2OCH_2CH_2)_2OH$ | 4,3 | 0 | 0 | 0,8 |
| dimère du bis(allylcarbonate) de diéthylèneglycol | 18,3 | 14,1 | 12,9 | 10,6 |
| trimère — | 5,7 | 2,0 | 1,8 | 1,0 |
| polymères | 3,6 | 2,8 | 4,7 | 7,5 |

Exemple 5

Dans un réacteur en verre Pyrex de 2 litres muni d'un agitateur, d'un thermomètre et d'un appareil de condensation on a introduit 1 420 g (10 moles) de carbonate d'allyle pur et 3,4 g (0,01 mole) de titanate de n.butyle. L'appareil est purgé à l'azote et, ensuite, la masse est portée à 150°C. Nous introduisons alors 106,1 g (1 mole) de diéthylèneglycol en 30 min en maintenant la température dans la zone considérée (±5°C). On poursuit par un palier de 1 heure à pression atmosphérique (durant lequel se produit une partie de la distillation de l'alcool allylique

formé) puis par un palier de 1 heure 30 min sous pression réduite (entre 450 et 90 torr) la température étant abaissée jusqu'à 110°C (pour la distillation du reste de l'alcoòl allylique).

On revient ensuite à la pression atmosphérique sous azote et après avoir refroidi la masse réactionnelle jusque vers 95-100°C le catalyseur est hydrolysé puis éliminé. On ajoute du charbon actif et on concentre sous pression réduite dans les mêmes conditions décrites à l'exemple 1.

Le rendement en produit isolé est de 93,6 %. La chromatographie liquide haute pression nous donne la composition suivante :

| | |
|---|---|
| $CH_2=CH-CH_2CO_3CH_2CH_2OCH_2CH_2OH$ | 0 % |
| bis(allylcarbonate) de diéthylèneglycol | 82,5 % |
| dimère du | 15,4 % |
| trimère du | 2,1 % |
| polymères | 0 % |

La viscosité du produit à 25°C est de 13,9 centistokes.

Exemple 6

Nous avons repris l'essai de l'exemple 5 à l'échelle supérieure (20 litres) avec :

-17,040 kg de carbonate d'allyle (120 moles)

- 1,273 kg de diéthylèneglycol (12 moles)

mais en utilisant 0,6 x $10^{-2}$ mole de titanate de n-butyle par mole de diéthylèneglycol soit au total 24,5 g de titanate de n-butyle (0,072 mole). Le tendement en produit isolé est 91,4 %. Sa composition est la suivante :

| | |
|---|---|
| $CH_2=CH-CH_2CO_3CH_2CH_2OCH_2CH_2OH$ | 0 % |
| bis(allylcarbonate) de diéthylèneglycol | 81,2 % |
| dimère du — | 14,9 % |
| trimère du — | 2,0 % |
| polymères | 1,9 % |

La teneur en eau est de 0,03 %

La viscosité à 25°C est de 14,8 centistokes.

Exemple 7

L'exemple 5 est repris à l'échelle d'un réacteur de 1 200 l avec le même taux de catalyseur (soit 1 % molaire par rapport au diéthylèneglycol engagé). Les charges sont cette fois de :

-921,1 kg (6 480 moles) pour la carbonate d'allyle

-68,75 kg (648 moles) pour le diéthylèneglycol),

-2,20 kg (6,48 moles) pour le titanate de n.butyle).

Le produit est isolé avec un rendement de 92,2 %.

La composition (toujours d'après la chromatographie liquide haute pression) est la suivante :

| | |
|---|---|
| $CH_2=CH-CH_2CO_3CH_2CH_2OCH_2CH_2OH$ | 0 % |
| bis(allylcarbonate) de diéthylèneglycol | 79,4 % |
| dimère du — | 15,4 % |
| trimère du — | 2,8 % |
| polymères | 2,4 % |

La couleur est égale à 5 APHA. La teneur en eau est de 0,03 %.

Exemple 8

Des essais de polymérisation de mélanges polymérisables selon l'invention ainsi que de mélanges dont la composition sort du cadre de l'invention ont été effectués.

L'initiateur choisi est le peroxyde de benzoyle (4,1 % en poids du mélange). Le mélange dans lequel est dissous l'initiateur est déshydraté sur sulfate de sodium. Après filtration, pour séparer l'agent déshydratant, le mélante est introduit, à l'aide d'une seringue, entre deux plaques de verre distantes de 3 mm et munies d'un joint adéquat. L'ensemble est placé dans une étuve ventilée dont la température est régulée de façon précise.

Le cycle de polymérisation est basé sur une montée en température progressive de 64 à 105°C en 17 heures. Il se termine par un palier de 1 heure à 105°C. L'éprouvette est ensuite refroidie lentement puis démoulée à température ambiante.

Nous comparons les différents polymères obtenus par :

-leur dureté pendulaire (en secondes au pendule PERSOZ) déterminée par une moyenne de plusieurs mesures pour chaque éprouvette,

-leur résistance à la rayure aux crayons de mines de différentes durés.

Les résultats obtenus avec des mélanges selon l'invention, des mélanges dont la composition sort du cadre de l'invention, un bis(allylcarbonate) de diéthylèneglycol distillé et un bis(allylcarbonate) de diéthylèneglycol du commerce sont rassemblés dans le tableau suivant.

**Tableau**

| numéro de référence | produit polymérisé | composition (% pondéraux) | | | | | dureté pendulaire (s) | rayure au crayon : |
|---|---|---|---|---|---|---|---|---|
| | | $CH_2=CH-CH_2CO_3CH_2CH_2OCH_2CH_2OH$ | bis(allyl-carbonate) de diéthylène-glycol | dimère | trimère | polymère | | |
| (1) | hors invention | 3,8 | 68 | 9,8 | 1,2 | 17,2 | 223 | 2 H |
| (2) | " | 1,3 | 70 | 13,2 | 2,1 | 13,4 | 225 | 3 H |
| (3) | " | 1,0 | 72,9 | 15,5 | 2,5 | 8,1 | 228 | 3 H |
| (4) | " | 2,0 | 76,5 | 14,5 | 2,0 | 5,0 | 226 | 3 H |
| (5) | distillé | 0 | 99,7 | 0 | 0 | 0 | 239 | 3 H |
| (6) | du commerce | 0 | 83,0 | 4,0 | 2,0 | 11,0 | 246 | 3 H Faible |
| (7) | selon l'invention | 0 | 80,1 | 15,8 | 2,5 | 1,6 | 247 | 4 H Faible |
| (8) | " | 0 | 81,2 | 14,9 | 2,0 | 1,9 | 246 | 4 H |
| (9) | " | 0 | 75,6 | 15,7 | 2,7 | 6,0 | 248 | 4 H Faible |

Les mélanges selon l'invention conduisent à des polymères possédant une dureté sensiblement améliorée. Cela se traduit notamment par une meilleure résistance à la rayure par une mine de crayon.

Exemple 9

Un échantillon de composition suivante :

| | |
|---|---|
| -bis(allylcarbonate) de diéthylèneglycol : | 76,5 % |
| -dimère du — | 15,8 % |
| -trimère du — | 2,6 % |
| -polymères | 5,1 % |

est soumis à un cycle de polymérisation à température constante (11 heures à 43°C) terminé par une cuisson par montée exponentielle en température jusqu'à 115-120°C en utilidant 5 % en poids de percarbonate de cyclohexyle.

Les éprouvettes, parfaitement incolores, ont une résistance à la rayure équivalente au produit du marché. Elles présentent, de plus, une parfaite résistance chocs et se comportent de façon excellente au test de résistance aux particules enflammées.

## REVENDICATIONS

1. Nouveaux mélanges polymérisables à base de bis(allylcarbonate) de diéthylèneglycol, caractérisés en ce qu'ils comportent

75 à 83 % en poids de bis(allylcarbonate) de diéthylèneglycol,

11 à 16 % en poids de dimère de ce bis(allylcarbonate) de diéthylèneglycol,

1 à 3 % en poids de trimère de ce bis(allylcarbonate) de diéthylèneglycol, et

0 à 7 % en poids de polymères ou copolymères de bis(allylcarbonate) de diéthylèneglycol,

lesdits mélanges contenant

-moins de 0,1 % d'eau

-moins de 1 % d'ester monosubstitué

2. Procédé de préparation de bis(allylcarbonate) de diéthylèneglycol et des mélanges contenant notamment 11 à 16 % en poids de dimère, 1 à 3 % en poids de trimère et 0 à 7 % en poids de polymère de ce produit, lesdits mélanges contenant moins de 0,1 % d'eau et moins de 1 % d'ester monosubstitué et étant directement utilisables pour la polymérisation, ledit procédé consistant dans une réaction de transestérification entre le carbonate d'allyle et le diéthylèneglycol, la réaction étant effectuée sous atmosphère inerte et en présence d'un excès de carbonate d'allyle et étant caractérisée en ce que

-la température de réaction est choisie entre 110 et 160°C, de préférence entre 130 et 155°C, avec élimination continue de l'alcool allylique formé, ladite élimination s'effectuant, en fin de réaction, à une température de 110 à 130°C et sous un vide partiel.

-le catalyseur, utilisé à raison de $0,2.10^{-2}$ à $2.10^{-2}$ mole par mole de diéthylèneglycol, est choisi parmi les esters de l'acide titanique et les acétylalétonates métalliques

puis, après élimination du catalyseur, on distille l'excès de carbonate pour récupérer le mélange polymérisable recherché.

3. Procédé selon la revendication 2 caractérisé en ce que le catalyseur est choisi parmi le titanate de n.butyle

et les acétylacétonates de titane et de zirconium.

4. Utilisation des nouveaux mélanges polymérisables selon la revendication 1, caractérisée en ce que ces mélanges sont polymérisés au moyen de catalyseurs radicalaires en vue de la préparation d'objets utilisables en optique.

Office européen des brevets

# RAPPORT DE RECHERCHE EUROPEENNE

0072325

Numéro de la demande

EP 82 40 1481

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| | --- | | C 08 F 218/16<br>C 07 C 69/96 //<br>(C 08 F 218/16<br>C 08 F 218/16<br>C 08 F 218/16 ) |
| A | FR-A-1 352 052 (RUTGERSWERKE AG)<br>----- | | |
| | | | **DOMAINES TECHNIQUES RECHERCHES (Int. Cl. 3)** |
| | | | C 08 F<br>C 07 C |

Le présent rapport de recherche a été établi pour toutes les revendications

| Lieu de la recherche | Date d'achèvement de la recherche | Examinateur |
|---|---|---|
| LA HAYE | 17-11-1982 | CAUWENBERG C.L.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82